# EUROPEAN PATENT APPLICATION

(11) **EP 0 679 390 A2**
(43) Date of publication of application: **02.11.1995**
(21) Application number: 95302619.2
(22) Date of filing: 19.04.1995
(51) Int. Cl.: A61K 9/12, A61K 9/70, A61L 25/00

(54) **Sprayable topical pharmaceutical compositions**

(30) Priority: 29.04.1994 GB 9408545
(71) Applicant: Zyma SA, CH-1260 Nyon (CH)
(72) Inventor: Hill, Ernest Arthur, Ambergate, Derbyshire, DE56 2HA (GB); Green, David Robert John, Wirksworth, Derbyshire, DE4 4EX (GB)
(74) Representative: Sharman, Thomas

(57) **Abstract**

A composition for the treatment of skin or a mucous membrane comprising a pharmaceutically active ingredient dissolved or dispersed in a gel-forming aqueous solution or dispersion of a gel-forming cellulosic polymer, said composition being sprayable to form, on contact with the skin or mucous membrane, an adherent cohesive mass.

## Description

This invention relates to compositions for the treatment of skin or a mucous membrane.

Certain infectious or disease states are characterised by the appearance of localised eruptions or sores on skin or mucous membrane surfaces which are often very painful when touched. Where viral or bacterial agents are responsible for this type of reaction, exudates from the affected areas can also be very infectious. Contact with these areas can significantly increase the risk of cross-infection to susceptible subjects. This is particularly characteristic, for example, of viral infections of the Herpes type. Small areas of skin or mucous membranes affected by this condition tend to be treated by localised application of a topical formulation intended to treat the underlying cause of the condition or the symptoms associated with the condition.

Examples of topical formulations conventionally used in the treatment of such conditions are creams, ointments, gels, lotions and paints. All existing commercial products of this type require physical contact between the affected area and whoever is applying the product, intimate contact being ensured by rubbing, spreading or similar means. Such contact is, however, often undesirable because of the likely discomfort suffered by the patient, or because of the risk of cross-contamination.

The present invention enables the need for such physical contact to be avoided.

Accordingly, the present invention provides a composition for the treatment of skin or a mucous membrane comprising a pharmaceutically active ingredient dissolved or dispersed in a gel-forming aqueous solution or dispersion of a gel-forming cellulosic polymer, said composition being sprayable to form, on contact with the skin or mucous membrane, an adherent cohesive mass.

The gel-forming solution or dispersion of the cellulosic polymer may be a solution or dispersion in water or a mixture of water together with another water-miscible solvent, such as ethylene glycol, propylene glycol or ethers thereof. Preferred cellulosic polymers include cellulose, alkyl celluloses such as methyl cellulose or ethyl cellulose, hydroxyalkyl celluloses such as hydroxyethyl cellulose or hydroxypropyl cellulose, hydroxyalkyl alkyl celluloses such as hydroxypropyl methyl cellulose or hydroxyethyl methyl cellulose, and carboxyalkyl celluloses such as carboxymethyl cellulose. In especially preferred compositions, the active ingredient is dissolved or dispersed in a gel-forming aqueous solution of methyl cellulose or hydroxyethyl methyl cellulose.

Compositions of the invention may optionally contain one or more other gel-forming organic polymers such as starch, gelatin or acrylic polymers and one or more other film-forming components, for example an aqueous solution or dispersion of a gum such as an alginate, arabic, guar, tragacanth or xanthan gum; a mixture of a mineral oil or vegetable oil with a viscosity modifier such as a high melting point wax or a colloidal inorganic filler such as colloidal silica, zinc oxide, kaolin or other clays; or a multiphasic dispersion, such as an oil/water emulsion, containing a surface active agent.

Depending on the properties required of the topical formulation of the invention, the pharmaceutically active ingredient may be, for example, an antiviral agent such as acyclovir, trifluridine, cytarabine, idoxuridine, 5¹-amino-5-iodo-2¹,5¹-dideoxyuridine, tromantadine, FIAC, ara-A, cyclaradine, Ganciclovir or 3,4-dihydroxybutylguanine (DHBG); an antiseptic, which may have virucidal activity with respect to certain viruses, such as chlorhexidine gluconate, Povidone iodine and other iodophors, p-chloro-m-xylenol (PCMX), n-hexylresorcinol, hexachlorophene (methylenebistrichlorophenol), dichlorophene (methylenebis-(4-chlorophenol), thiobis-(4,6-dichlorophenol), triclosan (trichlorohydroxydiphenyl ether), cetrimide, benzalkonium chloride, cetyl pyridinium chloride, laurolinium acetate or other related quaternary ammonium salt; a local anaesthetic, such as lignocaine, procaine, chloroprocaine, tetracaine, prilocaine, etidocaine or mepivacaine; or a mixture of two or more thereof.

Optional excipients which may also be incorporated in compositions of the invention include conventional ingredients of topical formulations, for example preservatives such as ethanol or an ester of p-hydroxybenzoic acid, such as the methyl, ethyl, n-propyl, n-butyl or benzyl ester or the sodium salt of the ester, surfactants such as polyoxyalkylene derivatives of alcohols and phenols and esters of such derivatives, astringents such as zinc sulphate, UV-absorbers and, particularly for compositions likely to be used in the perioral region, flavourings and fragrances.

For a given active ingredient, gel-forming polymer and other optional excipients, the amounts of these components required to give a composition which can be sprayed onto the skin or mucous membrane to form an adherent cohesive mass, i.e. a mass which will remain on the area to be treated and not drip or run off from that area, can readily be determined by experiment. The amount of pharmaceutically active ingredient is generally from 0.05 to 20%, more usually from 0.1 to 10%, by weight of the composition. The amount of gel-forming polymer is generally from 0.1 to 10%, more usually from 0.5 to 5%, by weight of the composition. The viscosity of the composition of the invention is generally about 0.01 to 20 Pas.

For administration to a patient, the composition of the invention is placed in a spray device, which may be a container fitted with a mechanical pump or a container pressurised with a propellant, for example a conventional aerosol propellant such as nitrogen, a hydrocarbon, e.g. butane, a chlorofluorocarbon or dimethyl ether. The spray device should preferably discharge a metered dose of the composition on actuation. In general, the metered dose may be up to 100µl per actuation, preferably from 10 to 50µl per actuation.

The composition of the invention is particularly useful for the treatment of cold sores and other conditions where cross-infection is likely or where the affected area is painful when touched.

The invention is illustrated by the following Examples, in which parts are by weight unless otherwise indicated.

### Example 1

A sprayable antiseptic/soothing gel for the treatment of cold sores is prepared by mixing chlorhexidine gluconate (1 part) with menthol (0.5 parts), methyl cellulose (0.5 parts), polyoxyethylene 20 sorbitan monolaurate surfactant (2 parts), polyethylene glycol mono(nonylphenyl) ether surfactant (2 parts), propylene glycol (10 parts) and purified water (84 parts). The resulting gel composition (80 parts) is placed in an aerosol spray device pressurised with dimethyl ether (20 parts) for application to affected skin in metered doses of 20µl per actuation.

### Example 2

A sprayable anti-herpes virus formulation for the treatment of cold sores is prepared by mixing acyclovir (5 parts), hydroxyethyl methyl cellulose (0.5 parts), polyoxyethylene 20 sorbitan monolaurate surfactant (2 parts), ethyl p-hydroxybenzoate preservative (0.1 part) and purified water (92.4 parts). The resulting composition (80 parts) is delivered into an aerosol spray device pressurised with dimethyl ether (20 parts) for application to affected skin in metered doses of 40µl per activation.

### Example 3

A sprayable antiviral formulation for the treatment of cold sores is prepared as described in Example 2, but replacing the acyclovir by idoxuridine (5 parts).

### Example 4

A sprayable antiviral formulation for the treatment of cold sores is prepared as described in Example 2, but replacing the acyclovir by tromantidine (1 part).

### Example 5

A sprayable composition with general antiseptic properties (including virucidal activity) is prepared by mixing cetrimide (0.5 part), hydroxyethyl methyl cellulose (0.5 part), polyoxyethylene 20 sorbitan monolaurate (2 parts), polyethyleneglycol mono(nonylphenyl) ether (2 parts) and purified water (95 parts). The resulting composition (80 parts) is delivered into an aerosol spray device pressurised with dimethyl ether (20 parts) for application to affected skin in metered doses of 25µl per actuation.

### Example 6

A sprayable composition with general antiseptic properties (including virucidal activity) is prepared as described in Example 5, but replacing the cetrimide by Povidone iodine (0.5 part).

### Example 7

A sprayable composition with general antiseptic properties (including virucidal activity) is prepared as described in Example 5, but replacing the cetrimide by benzalkonium chloride (0.1 part).

### Example 8

A sprayable antiseptic composition for the symptomatic relief of cold sores is prepared by mixing lignocaine hydrochloride (2.1 parts), cetrimide (0.5 part), zinc sulphate (1 part), menthol (0.5 part), hydroxyethyl methyl cellulose (0.75 part), polyoxyethylene 20 sorbitan monolaurate (2 parts), polyethyleneglycol mono-(nonylphenyl) ether (2 parts), propylene glycol (10 parts) and purified water (91.15 parts). The resulting composition (80 parts) is delivered into an aerosol spray device pressurised with dimethyl ether (20 parts) for application to affected skin in metered doses of 25µl per actuation. On actuating the spray device to spray a dose of the composition onto an area of skin, the composition forms a gel-like mass which adheres to the desired area of skin and does not run onto surrounding areas of skin.

## Claims

1. A composition for the treatment of skin or a mucous membrane comprising a pharmaceutically active ingredient dissolved or dispersed in a gel-forming aqueous solution or dispersion of a gel-forming cellulosic polymer, said composition being sprayable to form, on contact with the skin or mucous membrane, an adherent cohesive mass.

2. A composition according to Claim 1, in which the cellulosic polymer is an alkyl cellulose, a hydroxyalkyl cellulose, a hydroxyalkyl alkyl cellulose or a carboxyalkyl cellulose.

3. A composition according to claim 2, in which the active ingredient is dissolved or dispersed in a gel-forming aqueous solution of methyl cellulose or hydroxyethyl methyl cellulose.

4. A composition according to claim 1, 2 or 3, in which the pharmaceutically active ingredient is an antiviral agent, an antiseptic, a local anaesthetic or a mixture of two or more thereof.

5. A composition according to claim 1, 2 or 3, in which the pharmaceutically active ingredient is present in an amount of 0.1 to 10% by weight of the composition.

6. A composition according to claim 1, 2 or 3, in which the gel-forming polymer is present in an amount of 0.5 to 5% by weight of the composition.

7. A composition according to claim 1, 2 or 3, which has a viscosity of 0.01 to 20 Pas.

8. A composition according to claim 1, 2 or 3 which also contains a propellant.

9. A composition according to any of the preceding claims in a spray device which on actuation discharges a metered dose of the composition.

10. Use of a composition according to any of the preceding claims in the preparation of a medicament for the treatment of cold sores.
